# EUROPÄISCHE PATENTSCHRIFT

(11) **EP 3 824 067 B1**
(45) Veröffentlichungstag und Bekanntmachung des Hinweises auf die Patenterteilung: **31.08.2022**
(21) Anmeldenummer: 19748712.7
(22) Anmeldetag: 09.07.2019
(51) Int. Cl.: C12M 1/00

(54) **VORRICHTUNG ZUM ÜBERWACHEN EINES BIOLOGISCHEN PROZESSES IN EINEM FLÜSSIGEN MEDIUM**
DEVICE FOR MONITORING A BIOLOGICAL PROCESS IN A LIQUID MEDIUM
DISPOSITIF DE SURVEILLANCE D'UN PROCESSUS BIOLOGIQUE DANS UN MILIEU LIQUIDE

(30) Priorität: 18.07.2018 DE 102018117332
(43) Veröffentlichungstag der Anmeldung: 26.05.2021
(73) Patentinhaber: Hamilton Bonaduz AG, 7402 Bonaduz (CH)
(72) Erfinder: SCHÖNFUSS, Dirk, 7402 Bonaduz (CH); JUILLERAT, Frederic, 7402 Bonaduz (CH)
(74) Vertreter: PATERIS Patentanwälte PartmbB
(86) Internationale Anmeldenummer: PCT/EP2019/068437
(87) Internationale Veröffentlichungsnummer: WO 2020/016064

(56) Entgegenhaltungen:
- US-A1- 2011 187 388
- US-A1- 2014 322 786
- US-A1- 2018 002 653

## Beschreibung

### Gebiet der Erfindung

Die Erfindung betrifft eine Vorrichtung zum Überwachen eines biologischen Prozesses in einem flüssigen Medium. Die Erfindung betrifft weiter einen Bioreaktor mit einer Vorrichtung gemäß der Erfindung und einem Behälter. Die Erfindung betrifft ferner ein Verfahren zur Herstellung einer Vorrichtung zum Überwachen eines biologischen Prozesses in einem flüssigen Medium.

### Hintergrund der Erfindung

Die Kultivierung von Biomasse in einem Bioreaktor, der auch als Fermenter bezeichnet wird, erfordert eine ständige Überwachung der im Bioreaktor vorherrschenden Parameter wie beispielsweise der Zelldichte, des Sauerstoffgehalts oder des pH-Werts. Für die Überwachung dieser und anderer Parameter werden die Bioreaktoren mit entsprechenden Sensoren ausgestattet, die in das Innere des Bioreaktors hinein ragen.

Die Kultivierung der Biomasse erfolgt zunehmend in vorsterilisierten Einweg-Bioreaktoren aus flexiblem Kunststoff. Das Umwälzen der Biomasse im Betrieb des Bioreaktors wird durch ein Bewegen eines Behälters aus dem flexiblen Kunststoff, beispielsweise mittels einer Wippvorrichtung, erreicht.

Einweg-Bioreaktoren erfordern aufgrund ihrer Beschaffenheit und den damit verbundenen Betriebs-, Lager- und Sterilisierungsbedingungen anders gestaltete die Sensoren aufweisende Sensoranordnungen als herkömmliche Stahl-Fermenter. Die Sensoranordnungen sollen preiswert herzustellen sein, da sie nur einmal verwendet werden können, während sie in Bezug auf Messgenauigkeit und Stabilität mindestens genauso gut wie bei herkömmlichen Fermentern sein sollen. Beispiele für die Sensoren sind unter anderem für die optische pH- oder Sauerstoffmessung bekannt. Zudem sind Sensoren mit Elektroden für die elektrochemisch-kapazitive Biomasse- bzw. Zelldichte-Messung bekannt. Als Elektroden werden beispielsweise Metalldrähte verwendet.

Die Sensoranordnungen sind üblicherweise von Beginn an fest in den Einweg-Bioreaktor eingebaut und müssen den Bewegungen des Bioreaktors im Betrieb des Bioreaktors standhalten. Zudem müssen die Sensoranordnungen derart am Bioreaktor angebracht sein, dass weder Gase noch Flüssigkeiten aus dem Bioreaktor austreten können.

Um preiswert herstellbar zu sein, sollten die Sensoranordnungen für die Einweg-Bioreaktoren auch eine einfache Bauweise aufweisen. US 2011/187388 A offenbart eine Vorrichtung zum Überwachen eines biologischen Prozesses mit einer Sensoranordnung.

Es besteht daher ein Bedarf an Sensoranordnungen, die für Einweg-Bioreaktoren geeignet sind und eine einfache Bauweise aufweisen.

### Zusammenfassung der Erfindung

Die vorliegende Erfindung betrifft eine Vorrichtung zum Überwachen eines biologischen Prozesses in einem flüssigen Medium, mit einem Wandabschnitt, der eingerichtet ist, das Medium in einem Betrieb der Vorrichtung zu halten, einer Silanisierungsschicht, einem Kleber, und einer Sensoranordnung, wobei der Wandabschnitt ein Durchgangsloch aufweist, die Silanisierungsschicht unmittelbar auf dem Wandabschnitt und vollständig um das Durchgangsloch herum aufgebracht ist, die Sensoranordnung einen Träger aufweist, der eine Glasschicht aufweist, und der Kleber die Silanisierungsschicht mit der Glasschicht dicht verklebt.

Die Erfindung betrifft weiter einen Bioreaktor mit einer Vorrichtung gemäß der Erfindung und einem Behälter, in dem in einem Bioreaktorbetrieb des Bioreaktors das Medium anzuordnen ist, wobei der Behälter den Wandabschnitt aufweist, und wobei in dem Bioreaktorbetrieb der biologische Prozess durchzuführen ist.

Die Erfindung betrifft ferner ein Verfahren zur Herstellung einer Vorrichtung zum Überwachen eines biologischen Prozesses in einem flüssigen Medium, das Verfahren umfassend die Schritte:
a) Bereitstellen eines Wandabschnitts, der eingerichtet ist, das Medium in einem Betrieb der Vorrichtung zu halten, und einer Silanisierungsschicht, wobei der Wandabschnitt ein Durchgangsloch aufweist und die Silanisierungsschicht unmittelbar auf dem Wandabschnitt und vollständig um das Durchgangsloch herum aufgebracht ist,
b) Bereitstellen einer Sensoranordnung, wobei die Sensoranordnung einen Träger aufweist, der eine Glasschicht aufweist,
c) dichtes Verkleben der Silanisierungsschicht mit der Glasschicht mittels eines Klebers, und
d) Aushärten des Klebers.

### Kurzbeschreibung der Figuren

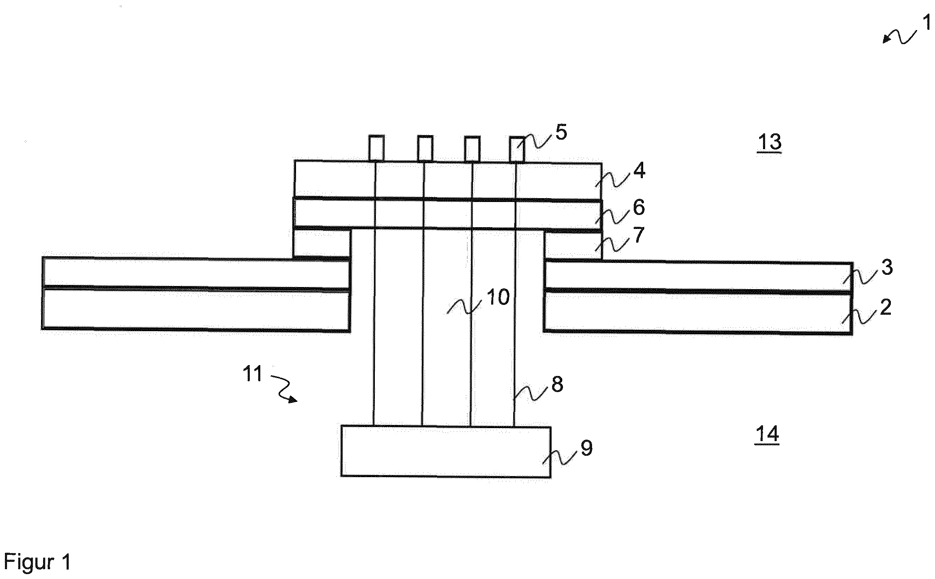

Figur 1 zeigt eine schematische Querschnittsdarstellung einer beispielhaften Ausführungsform der erfindungsgemäßen Vorrichtung, bei der das Substrat an einer zum Inneren eines Bioreaktors gewandten Seite des Wandabschnitts angebracht ist.

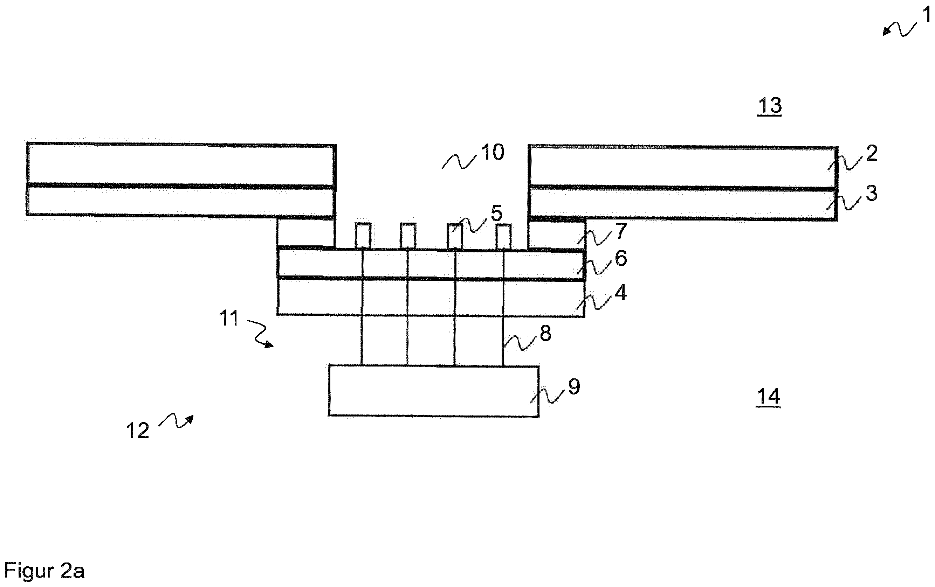

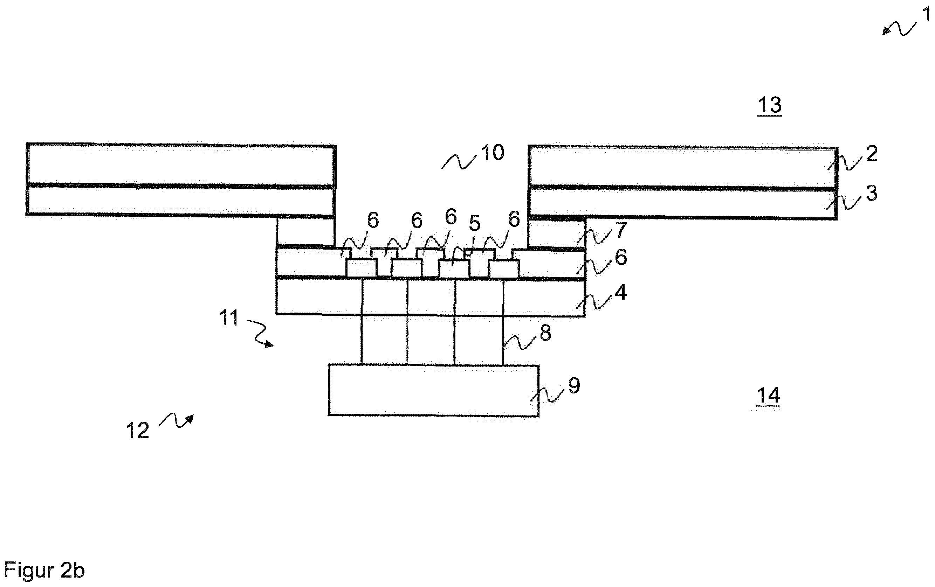

Figur 2 zeigt schematische Querschnittsdarstellungen von zwei beispielhaften Ausführungsformen der erfindungsgemäßen Vorrichtung, bei denen das Substrat an einer zum Äußeren des Bioreaktors gewandten Seite des Wandabschnitts angebracht ist. Die Glasschicht ist weiter zwischen den Elektroden und dem Substrat angeordnet (Figur 2a) oder zwischen den Elektroden und auf einem Teilbereich auf der dem Substrat abgewandten Oberfläche einer jeden Elektrode angeordnet, wobei der Teilbereich den Rand der dem Substrat abgewandten Oberfläche einer jeden Elektrode vollständig beinhaltet (Figur 2b).

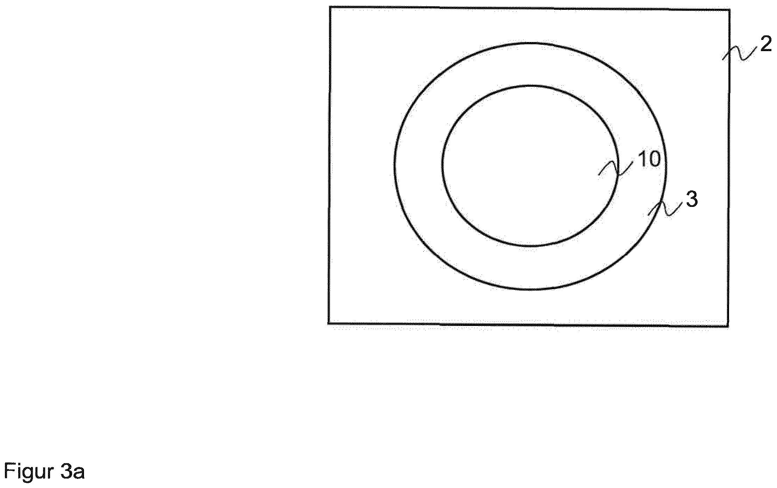

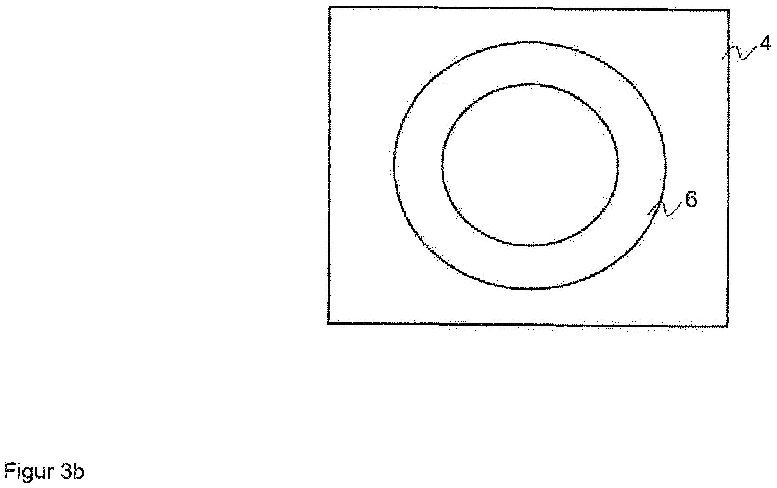

Figur 3 zeigt eine schematische Draufsicht auf den Wandabschnitt (Figur 3a) sowie auf das Substrat (Figur 3b).

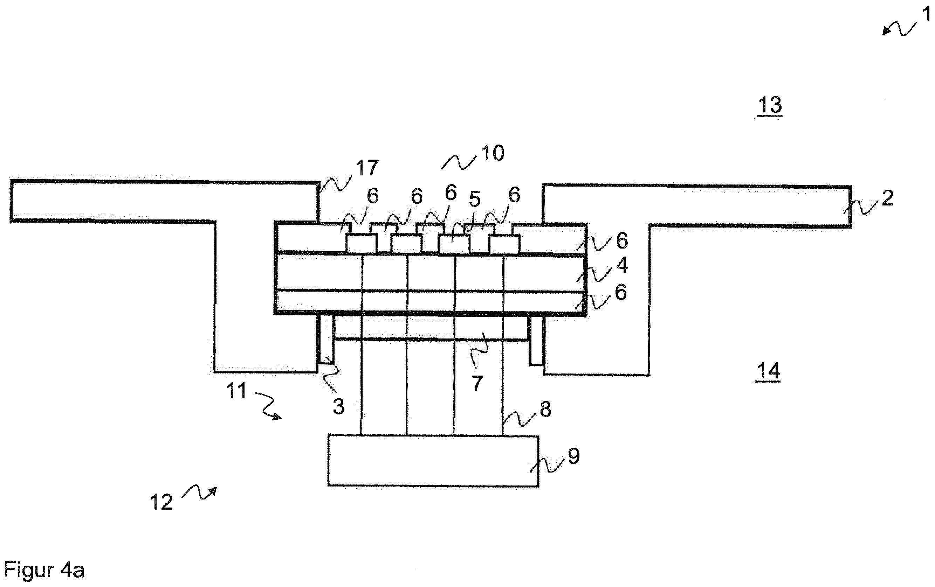

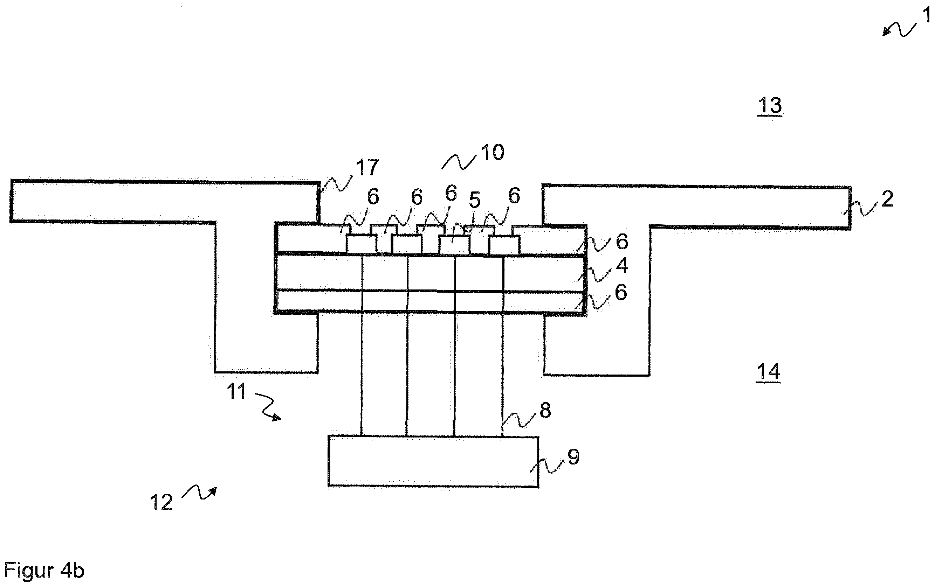

Figur 4 zeigt eine schematische Querschnittsdarstellung einer beispielhaften Ausführungsform der erfindungsgemäßen Vorrichtung, bei der der Wandabschnitt eine Durchgangslochoberfläche aufweist, die das Durchgangsloch begrenzt, und bei der die Glasschicht weiter zwischen den Elektroden und auf einem Teilbereich auf der dem Substrat abgewandten Oberfläche einer jeden Elektrode angeordnet ist, wobei der Teilbereich den Rand der dem Substrat abgewandten Oberfläche einer jeden Elektrode vollständig beinhaltet (Figur 4a). Figur 4 zeigt weiter ein Zwischenprodukt der Herstellung der in Figur 4a gezeigten Ausführungsform der erfindungsgemäßen Vorrichtung (Figur 4b).

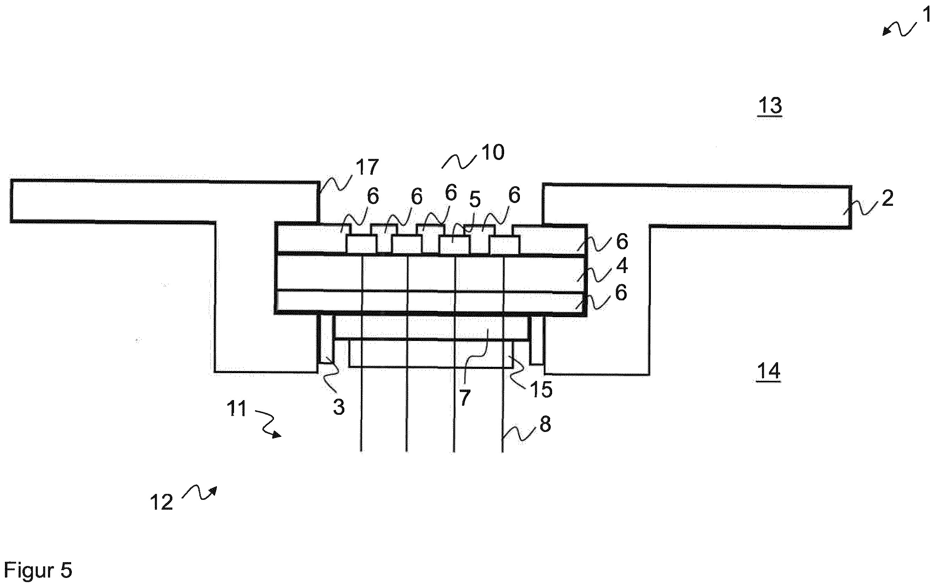

Figur 5 zeigt eine schematische Querschnittsdarstellung einer beispielhaften Ausführungsform der erfindungsgemäßen Vorrichtung, bei der die Sensoranordnung einen Kunststoffkörper aufweist, der die elektrisch leitfähigen Verbindungen zusammenhält.

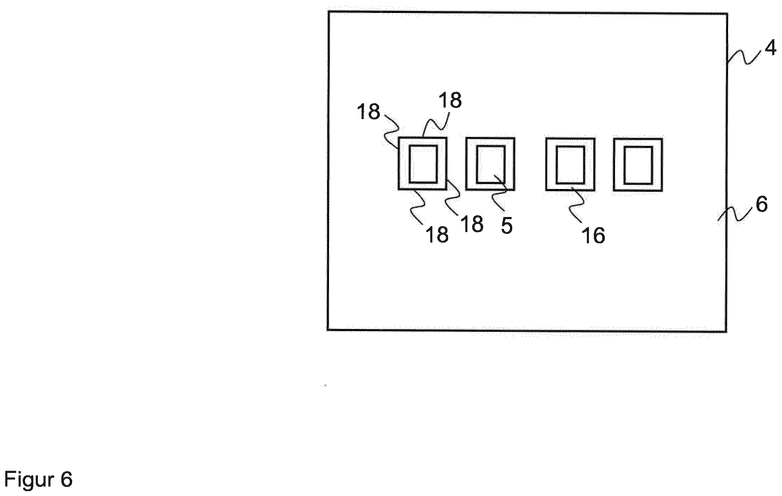

Figur 6 zeigt eine schematische Draufsicht auf das Substrat, auf dem die Glasschicht zwischen den Elektroden und auf einem Teilbereich auf der dem Substrat abgewandten Oberfläche einer jeden Elektrode angeordnet ist, wobei der Teilbereich den Rand der dem Substrat abgewandten Oberfläche einer jeden Elektrode vollständig beinhaltet.

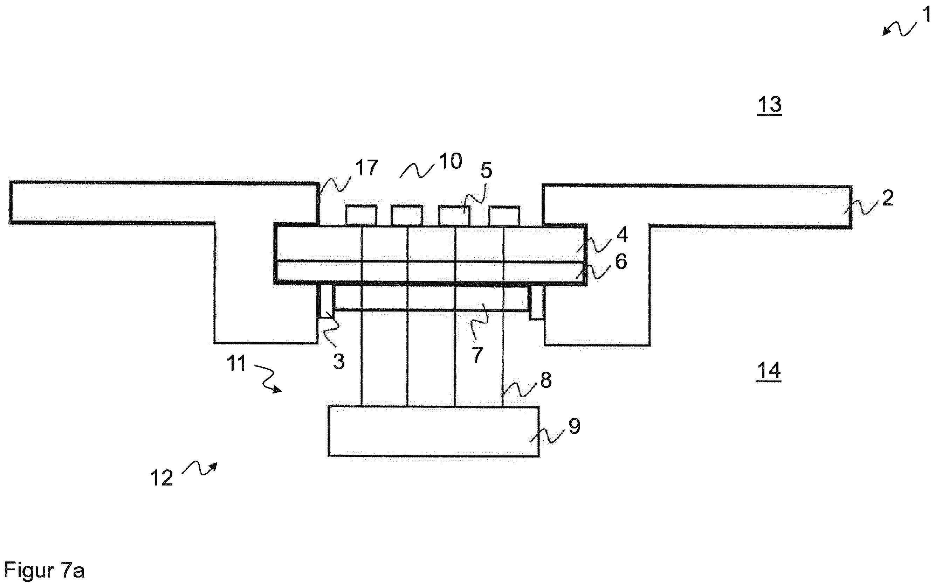

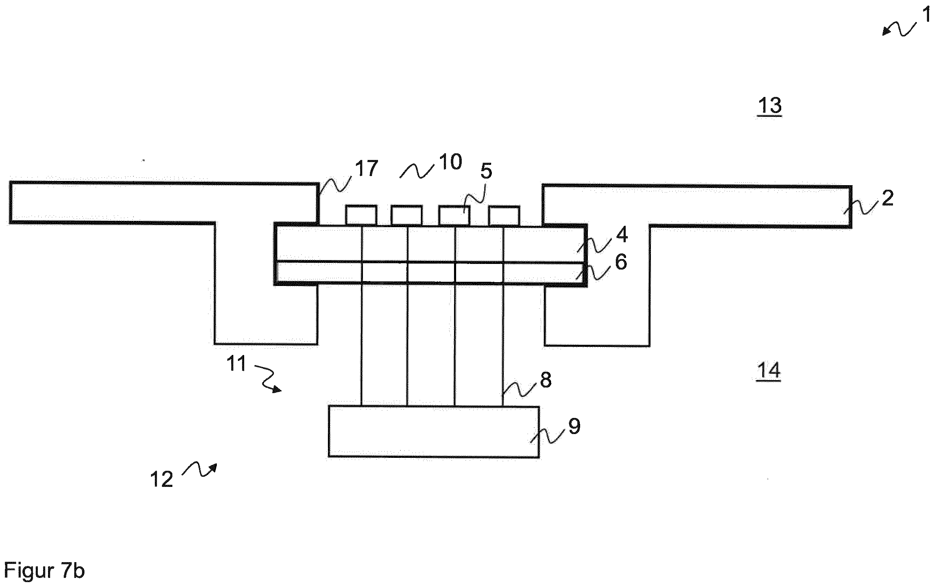

Figur 7 zeigt eine schematische Querschnittsdarstellung einer beispielhaften Ausführungsform der erfindungsgemäßen Vorrichtung, bei der der Wandabschnitt eine Durchgangslochoberfläche aufweist, die das Durchgangsloch begrenzt, und bei der die Glasschicht ausschließlich auf der zum Äußeren eines Bioreaktors gewandten Oberfläche des Substrats aufgebracht ist (Figur 7a). Figur 7 zeigt weiter ein Zwischenprodukt der Herstellung der in Figur 7a gezeigten Ausführungsform der erfindungsgemäßen Vorrichtung (Figur 7b).

### Detaillierte Beschreibung der Erfindung

In einem ersten Aspekt betrifft die Erfindung eine Vorrichtung zum Überwachen eines biologischen Prozesses in einem flüssigen Medium, mit einem Wandabschnitt, der eingerichtet ist, das Medium in einem Betrieb der Vorrichtung zu halten, einer Silanisierungsschicht, einem Kleber, und einer Sensoranordnung, wobei der Wandabschnitt ein Durchgangsloch aufweist, die Silanisierungsschicht unmittelbar auf dem Wandabschnitt und vollständig um das Durchgangsloch herum aufgebracht ist, die Sensoranordnung einen Träger aufweist, der eine Glasschicht aufweist, und der Kleber die Silanisierungsschicht mit der Glasschicht dicht verklebt.

Mit der erfindungsgemäßen Vorrichtung kann der biologische Prozess in dem flüssigen Medium überwacht werden. Der biologische Prozess kann beispielsweise die Produktion der Biomasse in einem Bioreaktor sein. Das flüssige Medium kann beispielsweise das Nährmedium von Zellen oder Bakterien sein, die als die Biomasse in dem Bioreaktor kultiviert werden. Das Überwachen des biologischen Prozesses wird durch die Sensoranordnung ermöglicht. Je nach Art eines in der Sensoranordnung eingesetzten Sensors können verschiedene Parameter wie beispielsweise Zelldichte, Sauerstoffgehalt oder pH-Wert erfasst und überwacht werden.

Die Vorrichtung weist den Wandabschnitt auf, der eingerichtet ist, das Medium in einem Betrieb der Vorrichtung zu halten. Der Wandabschnitt weist das Durchgangsloch auf, via das der Parameter ausgelesen werden kann. Beispielsweise kann dazu eine Verkabelung durch das Durchgangsloch geführt sein. Zusätzlich oder alternativ ist denkbar, dass eine elektromagnetische Strahlung via das Durchgangsloch geführt werden kann.

Die Vorrichtung weist weiter die Silanisierungsschicht auf, die unmittelbar auf dem Wandabschnitt und vollständig um das Durchgangsloch herum aufgebracht ist. Der Begriff "Silanisierung" wie hier verwendet bezeichnet eine chemische Anbindung einer Silanverbindung an eine Oberfläche. Die Silanisierungsschicht ist durch die Silanisierung der Oberfläche des Wandabschnitts gebildet und auf diese Weise unmittelbar auf dem Wandabschnitt aufgebracht.

Die Vorrichtung weist weiter die Sensoranordnung auf. Die Sensoranordnung weist den Träger auf, der die Glasschicht aufweist. Die Glasschicht des Trägers stellt eine Dichtfläche zwischen dem Wandabschnitt und dem Träger bereit. Zudem stellt der Träger mindestens einen Teil einer Messtechnik der Sensoranordnung bereit. Beispielsweise kann der Träger den Sensor tragen und/oder der Träger kann eingerichtet sein, die elektromagnetische Strahlung passieren zu lassen. Dadurch, dass der Träger die Dichtfläche und einen Teil der Messtechnik bereitstellt, ist der Träger ein Bauteil mit zwei Funktionen. Dadurch weist die Vorrichtung eine einfache Bauweise auf.

Die Vorrichtung weist weiter den Kleber auf. Der Kleber verklebt die Silanisierungsschicht dicht mit der Glasschicht. Das bedeutet, dass die Silanisierungsschicht derart mit der Glasschicht verklebt ist, dass das Medium nicht via das Durchgangsloch den Wandabschnitt passieren kann. Der Begriff "dicht" wie hier verwendet bezeichnet also flüssigkeitsdicht. Vorzugsweise ist die Verklebung der Silanisierungsschicht mit der Glasschicht nicht nur flüssigkeitsdicht, sondern zusätzlich auch gasdicht. Voraussetzung für die durch den Kleber erreichte Abdichtung ist die Kombination von Glasschicht, Kleber und Silanisierungsschicht. Diese Materialkombination führt dazu, dass der Kleber sowohl fest hält als auch dicht hält.

Da der Kleber die Silanisierungsschicht mit der Glasschicht verklebt und die Silanisierungsschicht vollständig um das Durchgangsloch des Wandabschnitts herum angeordnet ist, sind die Glasschicht und der Kleber ebenfalls vollständig um das Durchgangsloch des Wandabschnitts herum angeordnet.

Zusammengefasst weist die erfindungsgemäße Vorrichtung eine einfache Bauweise und sehr gute Abdichteigenschaften auf.

Die erfindungsgemäße Vorrichtung ist besonders geeignet, als Teil eines Bioreaktors die Produktion von Biomasse in dem Bioreaktor zu überwachen.

Für die Glasschicht können unterschiedliche Arten von Glas verwendet werden, die vorzugsweise in Abhängigkeit der Art des Sensors und/oder der vorgesehenen Anwendung der Vorrichtung ausgewählt sind. Die Glasschicht kann beispielsweise im Wesentlichen aus Quarzglas, auch Kieselglas genannt, bestehen. Das Quarzglas hat eine hohe chemische Beständigkeit und ist für Infrarot (IR)-Strahlung bis Ultraviolett (UV)-Strahlung durchlässig. Das Quarzglas ist besonders für optische Sensoren, die die elektromagnetische Strahlung im UV-Bereich detektieren, geeignet. Alternativ kann die Glasschicht beispielsweise im Wesentlichen aus Calciumfluorid bestehen. Calciumfluorid ist ebenfalls für IR- und UV-Strahlung durchlässig. Calciumfluorid ist besonders für die optischen Sensoren, die elektromagnetische Strahlung im IR-Bereich detektieren, geeignet. Kommerziell erhältliches Glas kann in Abhängigkeit vom Vormaterial und Fertigungsprozess geringe Mengen an Verunreinigungen und/oder Zusatzstoffen aufweisen, die die wesentlichen Eigenschaften des Glases nicht beeinträchtigen. Daher reicht es aus, wenn das Glas im Wesentlichen aus einer bestimmten Glasart besteht.

In einer bevorzugten Ausführungsform ist die Glasschicht der Silanisierungsschicht zugewandt angeordnet. Dadurch kann der Kleber eine relativ große Kontaktfläche sowohl mit der Glasschicht als auch mit der Silanisierungsschicht aufweisen. Dadurch verklebt der Kleber die Silanisierungsschicht mit der Glasschicht besonders dauerhaft.

In einer alternativen Ausführungsform weist der Wandabschnitt eine Durchgangslochoberfläche auf, die das Durchgangsloch begrenzt, wobei die Silanisierungsschicht auf der Durchgangslochoberfläche aufgebracht ist, und wobei die Sensoranordnung in dem Durchgangsloch angeordnet ist. In dieser Ausführungsform ist die Normale der Oberfläche der Glasschicht vorzugsweise im Wesentlichen senkrecht zu der Normalen der Oberfläche der Silanisierungsschicht angeordnet. Diese Anordnung erleichtert das Auftragen des Klebers, da die Kontaktstellen des Klebers mit der Glasschicht und der Silanisierungsschicht gut zugänglich sind.

In einer bevorzugten Ausführungsform ist der Kleber ein Silikonkleber oder ein Epoxidharzkleber. Die Erfinder haben festgestellt, dass der Silikonkleber oder der Epoxidharzkleber besonders dauerhaft und besonders dicht die Silanisierungsschicht mit der Glasschicht verkleben. Zudem halten diese Kleber einer Sterilisation mit Gammastrahlung stand.

Der Epoxidharzkleber kann beispielsweise Bisphenol-A-diglycidylether, Bisphenol-F-diglycidylether, Phenolnovolake, aliphatische Epoxide und/oder zykloaliphatische Epoxide aufweisen.

In einer bevorzugten Ausführungsform ist der Epoxidharzkleber ein Zweikomponentenkleber. In diesem Fall weist der Kleber ein Epoxidharz und einen Härter auf. Durch Vermischen des Epoxidharzes mit dem Härter unmittelbar vor der Anwendung des Klebers wird die Reaktion, durch die der Kleber aushärtet, gestartet. Der Härter kann beispielsweise ein Amin sein.

Die Erfinder haben verschiedene kommerziell erhältliche Silikonkleber und Epoxidharzkleber getestet. Dabei wurde mit dem Silikonkleber RS692-542 eine besonders dauerhafte und gute Dichtwirkung erzielt. Zudem wurden mit den Epoxidharzklebern Hysol RE2039 (Epoxidharz) mit Hysol HD6652 (Härter), EPO-TEK 301, EPO-TEK 301-2, Polytec EP 601 und Aremco-Bond 2310 eine besonders dauerhafte und gute Dichtwirkung erzielt.

In einer bevorzugten Ausführungsform ist der Zweikomponentenkleber EPO-TEK 301. Auch hier wurde eine besonders dauerhafte und gute Dichtwirkung erzielt.

In einer anderen Ausführungsform weist der Zweikomponentenkleber Hysol RE2039 als Epoxidharz und Hysol HD6652 als Härter auf. Auch hier wurde eine besonders dauerhafte und gute Dichtwirkung erzielt.

In einer bevorzugten Ausführungsform weist der Wandabschnitt einen Kunststoff oder ein Glas auf. Die Wände von Bioreaktoren bestehen häufig im Wesentlichen aus Glas oder aus Kunststoff. Der Wandabschnitt kann aus einem beliebigen Material bestehen, solange es möglich ist, die Silanisierungsschicht unmittelbar auf das Material des Wandabschnitts aufzubringen. Glas kann auf einfache und bekannte Weise silanisiert werden. Das Aufbringen der Silanisierungsschicht auf den Wandabschnitt kann durch direktes Silanisieren wie bei Glas oder aber durch eine Oberflächenaktivierung des Wandabschnitts mit anschließender Silanisierung, wie es beispielsweise beim Plasma-Silanisieren von Kunststoff der Fall ist, realisiert werden.

Einweg-Bioreaktoren sind in der Regel aus Kunststoff gefertigt. In einer besonders bevorzugten Ausführungsform weist der Wandabschnitt daher den Kunststoff auf. Dadurch kann der Wandabschnitt auf einfache Weise mit einer Wand des Einweg-Bioreaktors aus Kunststoff verschweißt werden oder selbst ein Abschnitt der Wand des Bioreaktors sein.

In einer weiter bevorzugten Ausführungsform weist der Kunststoff HDPE auf. Die Abkürzung "HDPE" bezeichnet Hart-Polyethylen (im Englischen "High Density Polyethylen"). HDPE wird auch als Polyethylen (PE) hoher Dichte bezeichnet. HDPE weist schwach verzweigte Polymerketten und daher eine hohe Dichte auf. Die Dichte von HDPE beträgt typischerweise von 0,94 g/cm³ bis 0,97 g/cm³.

In einer bevorzugten Ausführungsform weist der Wandabschnitt HDPE auf.

In einer besonders bevorzugten Ausführungsform besteht der Wandabschnitt im Wesentlichen aus HDPE. Kommerziell erhältliches HDPE kann geringe Mengen an Verunreinigungen und/oder Zusatzstoffen aufweisen, die die wesentlichen Eigenschaften des HDPE nicht beeinträchtigen. Daher reicht es aus, wenn der Wandabschnitt im Wesentlichen aus HDPE besteht. Da HDPE leicht zu verschweißen ist, kann der Wandabschnitt auf einfache Weise mit der Wand des Bioreaktors, die einen Kunststoff aufweist, verschweißt werden. In einer besonders bevorzugten Ausführungsform besteht der Wandabschnitt aus HDPE.

In einer bevorzugten Ausführungsform weist der Wandabschnitt HDPE auf und der Kleber ist der Silikonkleber oder der Epoxidharzkleber. Die Erfinder haben festgestellt, dass sich HDPE nach Aufbringen der Silanisierungsschicht mittels des Silikonklebers oder des Epoxidharzklebers dauerhaft haltbar und dicht mit der Glasschicht verkleben lässt.

In einer bevorzugten Ausführungsform ist der Wandabschnitt ein starres Bauteil. Das starre Bauteil ist vorzugsweise eine Scheibe, wie beispielsweise eine Verbindungsscheibe, die mit der Wand des Bioreaktors fest verbunden werden kann. Alternativ kann das starre Bauteil einen ersten Abschnitt und einen zweiten Abschnitt aufweisen, wobei der erste Abschnitt eingerichtet ist, mit einem anderen Teil der Wand des Bioreaktors fest verbunden zu werden, und der zweite Abschnitt die Durchgangslochoberfläche aufweist. Der erste Abschnitt und der zweite Abschnitt sind vorzugsweise im Wesentlichen senkrecht zueinander angeordnet. Durch das starre Bauteil kann die Vorrichtung besonders stabil in den Bioreaktor eingebaut werden. Dies ist insbesondere bei denjenigen Bioreaktoren von Vorteil, deren Behälter aus flexiblem Kunststoff sind und die im Betrieb des Bioreaktors zum Umwälzen der Biomasse bewegt werden.

In einer bevorzugten Ausführungsform ist der Wandabschnitt das starre Bauteil, das im Wesentlichen aus HDPE besteht, und der Kleber ist der Silikonkleber oder der Epoxidharzkleber.

Der Wandabschnitt kann auch von der Wand des Bioreaktors gebildet sein. Dadurch ist es nicht erforderlich, den Wandabschnitt fest mit der Wand des Bioreaktors zu verbinden, wodurch die Vorrichtung eine noch einfachere Bauweise aufweist. Diese Ausführungsform kommt insbesondere für diejenigen Bioreaktoren in Frage, deren Wände aus starrem Kunststoff oder aus einem anderen starren Material sind.

In einer bevorzugten Ausführungsform besteht der Träger aus der Glasschicht. Der Träger stellt in diesem Fall ein optisches Fenster bereit. Dies ermöglicht die Verwendung derjenigen Sensoranordnungen, die einen optischen Sensor aufweisen.

In einer bevorzugten Ausführungsform weist die Sensoranordnung den optischen Sensor auf, der einen Detektor aufweist, der eingerichtet ist, elektromagnetische Strahlung zu detektieren, die via das Durchgangsloch und die Glasschicht auf den Detektor trifft. Der optische Sensor kann zusätzlich eine Strahlungsquelle aufweisen, wobei der Detektor eingerichtet ist, von der Strahlungsquelle emittierte Strahlung nach deren Wechselwirkung mit dem Medium zu detektieren. Dabei können die Lichtquelle und der Detektor auf derselben Seite der Vorrichtung oder auf gegenüberliegenden Seiten der Vorrichtung angeordnet sein.

In einer bevorzugten Ausführungsform weist der Träger ein Substrat auf, auf dem die Glasschicht aufgebracht ist, und die Sensoranordnung weist mindestens einen der Sensoren auf, wobei der mindestens eine Sensor auf dem Substrat angeordnet ist. Die Glasschicht ist unmittelbar auf dem Substrat aufgebracht. Die Glasschicht kann auf der gesamten Oberfläche des Substrats, die der Silanisierungsschicht zugewandt ist, angeordnet sein. Alternativ kann die Glasschicht Aussparungen aufweisen, so dass es auf dem Substrat einen von der Glasschicht freien Bereich gibt. Der Sensor ist vorzugsweise auf einem von der Glasschicht freien Bereich des Substrats angeordnet. Zusätzlich oder alternativ ist denkbar, dass der Sensor auf einem von der Glasschicht freien Bereich des Substrats angeordnet ist und die Glasschicht weiter auf einem Teilbereich auf der dem Substrat abgewandten Oberfläche des Sensors angeordnet ist.

In einer bevorzugten Ausführungsform ist das Substrat ein Keramiksubstrat, wobei das Keramiksubstrat vorzugsweise Siliziumoxid und/oder Aluminiumoxid aufweist. In einer besonders bevorzugten Ausführungsform besteht das Keramiksubstrat aus Siliziumoxid und/oder Aluminiumoxid.

In einer bevorzugten Ausführungsform weist die Sensoranordnung einen Leitfähigkeitssensor auf, wobei der Leitfähigkeitssensor mindestens zwei Elektroden aufweist. Der Leitfähigkeitssensor ist eingerichtet, die elektrische Leitfähigkeit zwischen den zwei Elektroden zu messen. Die Elektroden sind auf dem Substrat aufgebracht. Die Sensoranordnung kann zudem Ableitbahnen aufweisen, die elektrisch leitfähig mit den Elektroden verbunden sind und via Durchkontaktierungsstellen durch den Träger hindurch geführt. Zudem kann die Sensoranordnung Kontaktstellen aufweisen, die elektrisch leitfähig mit den Ableitbahnen verbunden sind und dazu vorgesehen an eine Messelektronik elektrisch leitfähig angeschlossen zu werden. Die Sensoranordnung kann ferner einen Kunststoffkörper aufweisen, der die Ableitbahnen der Elektroden zusammenhält.

In einer bevorzugten Ausführungsform ist die Glasschicht weiter zwischen den Elektroden und dem Substrat angeordnet. Indem die Glasschicht auch zwischen den Elektroden und dem Substrat angeordnet ist, stellt die Glasschicht zusätzlich eine Abdichtung zwischen den Elektroden und dem Substrat bereit. Dadurch werden insbesondere die Durchkontaktierungsstellen in dem Substrat abgedichtet.

In einer alternativen Ausführungsform sind die Elektroden auf dem Substrat aufgebracht, und die Glasschicht ist weiter zwischen den Elektroden und auf einem Teilbereich auf der dem Substrat abgewandten Oberfläche einer jeden Elektrode angeordnet, wobei der Teilbereich den Rand der dem Substrat abgewandten Oberfläche einer jeden Elektrode vollständig beinhaltet. Der Begriff "Teilbereich" wie hier verwendet bezeichnet denjenigen Bereich der dem Substrat abgewandten Oberfläche einer Elektrode, der von der Glasschicht bedeckt ist. Auch in dieser Ausführungsform stellt die Glasschicht zusätzlich eine Abdichtung zwischen den Elektroden und dem Substrat bereit. Dadurch werden insbesondere die Durchkontaktierungsstellen in dem Substrat abgedichtet.

In einer bevorzugten Ausführungsform sind die Elektroden mittels Dünnschichttechnik oder Dickschichttechnik auf das Substrat aufgebracht.

In einer bevorzugten Ausführungsform weist die Sensoranordnung einen Leitfähigkeitssensor auf, wobei der Leitfähigkeitssensor vorzugsweise mindestens zwei Elektroden, weiter bevorzugt mindestens vier Elektroden, aufweist. Die Elektroden können beispielsweise auf der Glasschicht aufgebracht sein. Die Elektroden sind mit Ableitbahnen und Kontaktstellen für den Anschluss von Messelektronik auf der Rückseite der Glasschicht über Durchkontaktierungsstellen in der Glasschicht verbunden. Die Sensoranordnung kann zudem einen Kunststoffkörper aufweisen, der die Ableitbahnen der Elektroden zusammenhält.

Die Elektroden können beispielsweise für eine Leitfähigkeitsmessung, eine elektrochemisch-kapazitive Biomasse- bzw. Zelldichte-Messung oder eine ImpedanzMessung eingesetzt werden.

Die Elektroden können beispielsweise Metallelektroden, Wasserstoffelektroden oder Elektroden aus Graphit sein. In einer bevorzugten Ausführungsform sind die Elektroden Metallelektroden, vorzugsweise Platinelektroden.

In einer bevorzugten Ausführungsform weist die Sensoranordnung eine Kombination von unterschiedlichen Sensoren auf. Dadurch ist die Vorrichtung zum Überwachen von mehreren unterschiedlichen Parametern geeignet. Die Sensoranordnung kann beispielsweise einen optischen Sensor, einen Leifähigkeitssensor, einen Temperatursensor, einen Sauerstoffsensor und/oder einen pH-Wert-Sensor aufweisen.

In einer bevorzugten Ausführungsform weist die Sensoranordnung eines von einem Stecker und einer Steckdose auf, wobei das eine von dem Stecker und der Steckdose lösbar mit einem anderem von dem Stecker und der Steckdose verbindbar ist. Die Sensoranordnung kann zudem die Messelektronik und das andere von dem Stecker und der Steckdose aufweisen, das elektrisch leitfähig mit der Messelektronik verbunden ist. Dabei besteht im verbundenen Zustand via den Stecker und die Steckdose eine elektrische leitfähige Verbindung der Messelektronik mit dem Sensor, insbesondere mit den Elektroden. Die elektrische leitfähige Verbindung ermöglicht es, die Messelektronik zum Auslesen der von den Sensoren erfassten Parameter an die Sensoranordnung anzuschließen. Durch das Vorsehen des Steckers und der Steckdose ist die Messelektronik und das andere von dem Stecker und der Steckdose wiederverwendbar.

In einem zweiten Aspekt betrifft die Erfindung den Bioreaktor mit der Vorrichtung gemäß der Erfindung und einem Behälter, in dem in einem Bioreaktorbetrieb des Bioreaktors das Medium anzuordnen ist, wobei der Behälter den Wandabschnitt aufweist, und wobei in dem Bioreaktorbetrieb der biologische Prozess durchzuführen ist. Der Behälter weist die Wand des Bioreaktors auf.

In einer bevorzugten Ausführungsform ist der Träger an einer zum Äußeren des Behälters gewandten Seite des Wandabschnitts angebracht. Die Vorrichtung lässt sich auf diese Weise einfach an den Bioreaktor anbringen.

In einer bevorzugten Ausführungsform ist der Träger an einer zum Inneren des Behälters gewandten Seite des Wandabschnitts angebracht. Auf diese Weise stellt der Träger mehr Fläche für das Anbringen des Sensors oder mehrerer der Sensoren bereit. Der Sensor kann auf der gesamten zum Inneren des Behälters gewandten Seite des Trägers angebracht werden. Dagegen kann der Sensor bei einem Anbringen des Trägers an einer zum Äußeren des Behälters gewandten Seite des Wandabschnitts nur auf dem Abschnitt des Trägers, der unmittelbar an dem Durchgangsloch angeordnet ist, angebracht werden.

In einer bevorzugten Ausführungsform ist der Wandabschnitt ein starres Bauteil. Das starre Bauteil ist vorzugsweise eine Scheibe, wie beispielsweise eine Verbindungsscheibe, die mit einem anderen Teil der Wand des Bioreaktors fest verbunden werden kann. Alternativ kann das starre Bauteil einen ersten Abschnitt und einen zweiten Abschnitt aufweisen, wobei der erste Abschnitt eingerichtet ist, mit einem anderen Teil der Wand des Bioreaktors fest verbunden zu werden, und der zweite Abschnitt die Durchgangslochoberfläche aufweist. Der erste Abschnitt und der zweite Abschnitt sind vorzugsweise im Wesentlichen senkrecht zueinander angeordnet. Durch das starre Bauteil kann die Vorrichtung besonders stabil in den Bioreaktor eingebaut werden. Dies ist insbesondere bei einem Behälter, der im Wesentlichen aus einem flexiblem Kunststoff besteht, von Vorteil, da ein derartiger Bioreaktor im Bioreaktorbetrieb bewegt wird, um die Biomasse umzuwälzen.

In einer bevorzugten Ausführungsform weist der Behälter einen Plastiksack auf, der vorzugsweise flexibel ist. Der Plastiksack weist ein weiteres Durchgangsloch auf, das dem Durchgangsloch des Wandabschnitts entspricht.

Der Plastiksack kann Polyethylenterephthalat (PET), Polyethylen niedriger Dichte (LDPE), Polyvinylacetat (PVA), Polyvinylchlorid (PVC) und/oder Polypropylen (PP) aufweisen.

In einer bevorzugten Ausführungsform ist der Wandabschnitt ein starres Bauteil und der Behälter weist einen Plastiksack auf, der mit dem starren Bauteil fest verbunden ist, wobei der Plastiksack vorzugsweise flexibel ist. Die feste Verbindung zwischen dem Plastiksack und dem starren Bauteil kann beispielsweise durch Verschweißen des Plastiksacks mit dem starren Bauteil erreicht werden. Die Kombination eines flexiblen Plastiksacks mit einem starren Bauteil als Wandabschnitt ist besonders vorteilhaft. Die Vorrichtung ist auf diese Weise stabil in den Bioreaktor eingebaut, so dass sie den Bewegungen des Bioreaktors im Betrieb des Bioreaktors standhält.

In einer bevorzugten Ausführungsform ist der Bioreaktor ein Einweg-Bioreaktor. Einweg-Bioreaktoren haben den Vorteil, dass aufwändige Reinigungs- und Sterilisierungsprozesse, die vor einer erneuten Inbetriebnahme eines mehrfach verwendbaren Bioreaktors erforderlich sind, entfallen. Dadurch kann der Einweg-Bioreaktor auch Materialien aufweisen, die solchen Reinigungs- und Sterilisierungsprozessen nicht standhalten würden.

In einem dritten Aspekt betrifft die Erfindung ein Verfahren zur Herstellung einer Vorrichtung zum Überwachen eines biologischen Prozesses in einem flüssigen Medium, das Verfahren umfassend die Schritte:
a) Bereitstellen eines Wandabschnitts, der eingerichtet ist, das Medium in einem Betrieb der Vorrichtung zu halten, und einer Silanisierungsschicht, wobei der Wandabschnitt ein Durchgangsloch aufweist und die Silanisierungsschicht unmittelbar auf dem Wandabschnitt und vollständig um das Durchgangsloch herum aufgebracht ist,
b) Bereitstellen einer Sensoranordnung, wobei die Sensoranordnung einen Träger aufweist, der eine Glasschicht aufweist,
c) dichtes Verkleben der Silanisierungsschicht mit der Glasschicht mittels eines Klebers, und
d) Aushärten des Klebers.

Für das Verkleben der Silanisierungsschicht mit der Glasschicht ist es zweckmä-βig, den Wandabschnitt relativ zu der Sensoranordnung derart anzuordnen, dass die Silanisierungsschicht eine räumliche Nähe zu der Glasschicht aufweist.

Da die Silanisierungsschicht vollständig um das Durchgangsloch des Wandabschnitts herum angeordnet ist, erfolgt das Verkleben der Silanisierungsschicht mit der Glasschicht ebenfalls vollständig um das Durchgangsloch des Wandabschnitts herum.

In einer bevorzugten Ausführungsform ist der Kleber ein Silikonkleber oder ein Epoxidharzkleber.

In einer bevorzugten Ausführungsform ist der Epoxidharzkleber ein Zweikomponentenkleber.

Die Erfinder haben verschiedene kommerziell erhältliche Silikonkleber und Epoxidharzkleber getestet. Dabei hat sich beispielsweise der Silikonkleber RS692-542 als besonders geeignet heraus gestellt. Zudem haben sich die Epoxidharzkleber Hysol RE2039 (Epoxidharz) mit Hysol HD6652 (Härter), EPO-TEK 301, EPO-TEK 301-2, Polytec EP 601 und Aremco-Bond 2310 als besonders geeignet heraus gestellt.

In einer bevorzugten Ausführungsform ist der Zweikomponentenkleber EPO-TEK 301.

In einer anderen Ausführungsform weist der Zweikomponentenkleber Hysol RE2039 als Epoxidharz und Hysol HD6652 als Härter auf.

In einer bevorzugten Ausführungsform weist der Wandabschnitt einen Kunststoff oder ein Glas, vorzugsweise einen Kunststoff, weiter bevorzugt HDPE auf.

In einer bevorzugten Ausführungsform wird in Schritt a) die Silanisierungsschicht durch Silanisieren mit einem Silanisierungsreagenz auf den Wandabschnitt aufgebracht. Das Silanisieren kann ein direktes Silanisieren oder ein Silanisieren nach vorhergehender Oberflächenaktivierung des Wandabschnitts, beispielsweise durch Plasma-Aktivierung, sein. HDPE und andere Kunststoffe können beispielsweise nach einer Oberflächenaktivierung mittels Plasma (Plasma-Aktivierung) silanisiert werden. Der Begriff "Plasma-Aktivierung" wie hier verwendet bezeichnet ein Verfahren, bei dem mittels Plasma OH-Gruppen in die Oberfläche eines Kunststoffs eingebaut werden. Die OH-Gruppen reagieren während des Silanisierens mit dem Silanisierungsreagenz. Der Begriff "Plasma-Silanisieren" wie hier verwendet bezeichnet ein Verfahren zum Silanisieren einer Oberfläche, bei dem zunächst eine Aktivierung der Oberfläche mittels Plasma und anschließend ein Silanisieren der aktivierten Oberfläche mit einem Silanisierungsreagenz durchgeführt wird. Das Plasma-Silanisieren wird insbesondere bei einem Wandabschnitt, der einen Kunststoff aufweist, eingesetzt.

Silanisierungsreagenzien sind bekannt. Das Silanisierungsreagenz kann beispielsweise 3-(Triethoxysilyl)propylisocyanat, Methoxytrimethylsilan, Ethoxytrimethylsilan, Dimethoxydimethylsilan, Trimethoxymethylsilan, Trimethoxyethylsilan, Trimethoxypropylsilan, Trimethoxyphenylsilan, (Trimethylsilyl)methanol, (Trimethylsilyl)isocyanat, (3-Aminopropyl)triethoxysilan, Isopropoxytrimethylsilan oder Dimethoxymethylphenylsilan sein. Das Silanisierungsreagenz kann auch eine Mischung von zwei oder mehr Verbindungen sein.

In einer bevorzugten Ausführungsform weist das Silanisierungsreagenz 3-(Triethoxysilyl)propylisocyanat auf.

In einer bevorzugten Ausführungsform wird in Schritt b) die Glasschicht mittels Siebdruck einer Glaspaste auf den Träger aufgebracht und eingebrannt. Glaspasten sind bekannt. Durch das Aufbringen der Glaspaste können zugleich die Durchkontaktierungsstellen in dem Träger abgedichtet werden.

In einer bevorzugten Ausführungsform wird vor Schritt c), vor Schritt d) oder nach Schritt d) ein Stecker oder eine Steckdose bereitgestellt und mit dem Wandabschnitt fest verbunden. Die feste Verbindung zwischen dem Stecker oder der Steckdose und dem Wandabschnitt kann beispielsweise durch Verschweißen des Steckers oder der Steckdose mit dem Wandabschnitt erreicht werden.

Das Aufbringen des Klebers kann beispielsweise durch Gießen oder durch ein Auftragen des Klebers mittels einer Schablone und eines Rollers erfolgen.

In einer bevorzugten Ausführungsform wird in Schritt c) der Kleber auf die Silanisierungsschicht und auf die Glasschicht aufgebracht.

Das Aushärten des Klebers kann beispielsweise bei Raumtemperatur, also bei etwa bei 23°C, oder bei einer höheren Temperatur durchgeführt werden. Bei höheren Temperaturen verkürzt sich die Dauer des Aushärtevorgangs und es wird üblicherweise eine höhere Festigkeit des ausgehärteten Klebers erreicht.

In einer bevorzugten Ausführungsform wird Schritt d) für 24 Stunden bei etwa 23°C oder für 1 Stunde bei 65°C durchgeführt.

Der Silikonkleber RS692-542 wird vorzugsweise bei etwa 23°C ausgehärtet.

Die Epoxidharzkleber Hysol RE2039 (Epoxidharz) mit Hysol HD6652 (Härter), EPO-TEK 301, EPO-TEK 301-2, Polytec EP 601 und Aremco-Bond 2310 werden vorzugsweise bei 65°C ausgehärtet.

In einer bevorzugten Ausführungsform ist der Zweikomponentenkleber EPO-TEK 301 und Schritt d) wird für 1 Stunde bei 65°C durchgeführt.

In einer anderen Ausführungsform weist der Zweikomponentenkleber Hysol RE2039 als Epoxidharz und Hysol HD6652 als Härter auf und Schritt d) wird für 1 Stunde bei 65°C durchgeführt.

Im Folgenden werden bevorzugte Ausführungsbeispiele einer erfindungsgemäßen Vorrichtung anhand der beigefügten schematischen Zeichnungen erläutert.

Die Figuren 1, 2a und 2b zeigen jeweils eine Querschnittsdarstellung einer bevorzugten Ausführungsform der erfindungsgemäßen Vorrichtung 1. Wie es aus den Figuren 1, 2a und 2b ersichtlich ist, weist die Vorrichtung 1 einen Wandabschnitt 2, eine Silanisierungsschicht 3, einen Kleber 7, eine Glasschicht 6, ein Substrat 4, vier Elektroden 5, vier elektrisch leitfähige Verbindungen 8 und eine Steckdose 9 auf. Das Substrat 4, die Elektroden 5, die Glasschicht 6, die elektrisch leitfähigen Verbindungen 8 und die Steckdose 9 bilden Teile einer Sensoranordnung 12 der Vorrichtung 1. Der Wandabschnitt 2 besteht aus HDPE und weist ein Durchgangsloch 10 auf. Die Silanisierungsschicht 3, der Kleber 7 und die Glasschicht 6 sind vollständig um das Durchgangsloch 10 herum angeordnet. Die Silanisierungsschicht 3 ist unmittelbar auf dem Wandabschnitt 2 aufgebracht und mittels des Klebers 7 dicht mit der Glasschicht 6 verklebt. Die Glasschicht 6 ist unmittelbar auf dem Substrat 4 aufgebracht. Das Substrat 4 ist ein Keramiksubstrat, das aus Siliziumoxid besteht. Auf dem Substrat 4 sind die Elektroden 5 aufgebracht, die einen Sensor bereitstellen. Die Elektroden 5 sind Platinelektroden. Die Elektroden 5 sind via elektrisch leitfähige Verbindungen 8 mit einer Steckdose 9 verbunden. Die elektrisch leitfähigen Verbindungen 8 können Metallstifte sein, die einen Stecker 11 bilden. Alternativ kann es sich bei den elektrisch leitfähigen Verbindungen 8 um Drähte handeln.

Alternativ kann die Glasschicht 6 ohne das Substrat 4 vorliegen, wobei die Elektroden 5 in diesem Fall auf der Glasschicht 6 angeordnet sind.

In Figur 1 ist das Substrat 4 an einer zum Inneren 13 eines Bioreaktors gewandten Seite des Wandabschnitts 2 angebracht.

In den Figuren 2a und 2b ist das Substrat 4 dagegen an einer zum Äußeren 14 eines Bioreaktors gewandten Seite des Wandabschnitts 2 angebracht.

In Figur 2a ist die Glasschicht 6 weiter zwischen den Elektroden 5 und dem Substrat 4 angeordnet. Die Elektroden 5 sind von der Glasschicht 6 frei.

Im Unterschied zu Figur 2a ist die Glasschicht 6 in Figur 2b weiter zwischen den Elektroden 5 und in einem Bereich entlang des gesamten Umfangs einer jeden Elektrode 5 auf den Elektroden 5 angeordnet. Dadurch sind die Elektroden 5 teilweise von der Glasschicht 6 bedeckt. Jede Elektrode 5 weist in ihrer Mitte einen von der Glasschicht 6 freien Bereich auf, damit die Elektroden 5 ihre Funktion, beispielsweise für eine Leitfähigkeitsmessung, ausüben können.

Figur 3a zeigt eine Draufsicht auf den Wandabschnitt 2. Es ist ersichtlich, dass der Wandabschnitt 2 ein Durchgangsloch 10 aufweist und die Silanisierungsschicht 3 vollständig um das Durchgangsloch 10 herum angeordnet ist.

Figur 3b zeigt eine Draufsicht auf das Substrat 4. Es ist ersichtlich, dass die Glasschicht 6 auf dem Substrat 4 in einem Bereich angeordnet ist, der in der zusammengebauten Vorrichtung 1 vollständig um das Durchgangsloch 10 herum angeordnet ist. Alternativ kann die Glasschicht 6 auf der gesamten Oberfläche des Substrats 4, die der Silanisierungsschicht 3 zugewandt ist, angeordnet sein.

Die in den Figuren 1, 2a und 2b gezeigten Ausführungsformen der erfindungsgemäßen Vorrichtung 1 können wie folgt hergestellt werden:
Zunächst wird der Wandabschnitt 2, der aus HDPE besteht und das Durchgangsloch 10 aufweist, bereitgestellt. Dann wird die Silanisierungsschicht 3 durch Plasma-Silanisieren unmittelbar auf den Wandabschnitt 2 aufgebracht. Für die Plasma-Silanisierung werden zwei offene Glasbehälter jeweils mit ca. 0,1 ml 3-(Triethoxysilyl)propylisocyanat gefüllt und zusammen mit dem Wandabschnitt 2 in eine Vakuumkammer einer sogenannten Plasma-Silanisierungsanlage eingelegt. Dabei wird der Wandabschnitt 2 zwischen zwei Elektroden in der Vakuumkammer angeordnet. Die Vakuumkammer wird dann bis ca. 0,2 mbar abgepumpt und Plasma wird mittels eines Generators zwischen den zwei Elektroden in der Vakuumkammer gezündet. Der Wandabschnitt 2 wird für zwei Minuten mit Plasma behandelt und silanisiert. Anschließend wird die Vakuumkammer belüftet.

Im nächsten Schritt wird das Substrat 4 mit den Elektroden 5, der Glasschicht 6 und den elektrisch leitfähigen Verbindungen 8 bereitgestellt. Zudem wird der Stecker 11 bereitgestellt. Der Wandabschnitt 2, das Substrat 4 und der Stecker 11 werden zusammengeführt, indem der Wandabschnitt 2 auf einen Tisch gelegt wird, das Substrat 4 darauf angeordnet wird und schließlich der Stecker 11 darauf angeordnet wird. Der Stecker 11 wird nun mit dem Wandabschnitt 2 verschweißt. Das Glasschicht 6 des Substrats 4 und die Silanisierungsschicht 3 des Wandabschnitts 2 werden dabei nicht fest miteinander verbunden. Das Substrat 4 kann insbesondere noch bewegt werden. Das Substrat 4 wird zum Auftragen des Klebers 7 auf die Glasschicht 6 und die Silanisierungsschicht 3 etwas von dem Wandabschnitt 2 abgehoben. Nach dem Auftragen des Klebers 7 werden das Substrat 4 und der Wandabschnitt 2 derart zueinander positioniert, dass der Kleber 7 die Silanisierungsschicht 3 mit der Glasschicht 6 verklebt. Schließlich wird der Kleber 7 bei 65°C für 1 Stunde ausgehärtet.

Figur 4a zeigt eine Querschnittsdarstellung einer bevorzugten Ausführungsform der erfindungsgemäßen Vorrichtung 1. Die Vorrichtung 1 weist einen Wandabschnitt 2, eine Silanisierungsschicht 3, einen Kleber 7, eine Glasschicht 6, ein Substrat 4, eine Mehrzahl von Elektroden 5, eine Mehrzahl von elektrisch leitfähigen Verbindungen 8 und eine Steckdose 9 auf. Die Vorrichtung 1 kann beispielsweise vier Elektroden 5 und vier elektrisch leitfähige Verbindungen 8 aufweisen. Der Wandabschnitt 2 besteht aus HDPE und weist ein Durchgangsloch 10 auf. Es ist ersichtlich, dass der Wandabschnitt 2 eine Durchgangslochoberfläche 17 aufweist, die das Durchgangsloch 10 begrenzt. Das Substrat 4, die Elektroden 5, die Glasschicht 6, die elektrisch leitfähigen Verbindungen 8 und die Steckdose 9 bilden Teile einer Sensoranordnung 12 der Vorrichtung 1. Die Sensoranordnung 12 ist in dem Durchgangsloch 10 angeordnet. Die Sensoranordnung 12 ist im Wandabschnitt 2 durch eine umlaufende Nut im Wandabschnitt 2 fixiert. Dadurch kann sich die Sensoranordnung 12 nicht relativ zu dem Wandabschnitt 2 bewegen. Die Silanisierungsschicht 3 ist unmittelbar auf der Durchgangslochoberfläche 17 des Wandabschnitts 2 aufgebracht und vollständig um das Durchgangsloch 10 herum angeordnet. Die Glasschicht 6 ist unmittelbar auf dem Substrat 4 aufgebracht. Die Glasschicht 6 ist auf der gesamten zum Äußeren 14 eines Bioreaktors gewandten Oberfläche des Substrats 4 und weiter auf der zum Inneren 13 eines Bioreaktors gewandten Oberfläche des Substrats 4 aufgebracht. Auf der zum Inneren 13 eines Bioreaktors gewandten Oberfläche des Substrats 4 ist die Glasschicht 6 zwischen den Elektroden 5 und auf einem Teilbereich 16 auf der dem Substrat 4 abgewandten Oberfläche einer jeden Elektrode 5 angeordnet, wobei der Teilbereich 16 den Rand 18 der dem Substrat 4 abgewandten Oberfläche einer jeden Elektrode 5 vollständig beinhaltet. Dadurch sind die Elektroden 5 teilweise von der Glasschicht 6 bedeckt. Jede Elektrode 5 weist in ihrer Mitte einen von der Glasschicht 6 freien Bereich auf, damit die Elektroden 5 ihre Funktion, beispielsweise für eine Leitfähigkeitsmessung, ausüben können. Die Normale der zum Äußeren 14 eines Bioreaktors gewandten Oberfläche der Glasschicht 6 ist senkrecht zu der Normalen der Oberfläche der Silanisierungsschicht 3 angeordnet. Die Silanisierungsschicht 3 ist mittels des Klebers 7 dicht mit dem zum Äußeren 14 eines Bioreaktors gewandten Bereich der Glasschicht 6 verklebt. Der Kleber 7 ist vollständig um das Durchgangsloch 10 herum angeordnet. Der Kleber 7 ist auf den gesamten zum Äußeren 14 eines Bioreaktors gewandten Bereich der Glasschicht 6 aufgebracht. Alternativ kann der Kleber 7 nur in einem Abschnitt des zum Äußeren 14 eines Bioreaktors gewandten Bereichs der Glasschicht 6 aufgebracht sein, wobei der Abschnitt vollständig um das Durchgangsloch 10 herum angeordnet ist. Das Substrat 4 ist ein Keramiksubstrat, das Siliziumoxid aufweist. Auf dem Substrat 4 sind die Elektroden 5 aufgebracht, die einen Sensor bilden. Die Elektroden 5 können beispielsweise Platinelektroden sein. Die Elektroden 5 sind via elektrisch leitfähige Verbindungen 8 mit einer Steckdose 9 verbunden. Die elektrisch leitfähigen Verbindungen 8 können Metallstifte sein, die einen Stecker 11 bilden. Alternativ kann es sich bei den elektrisch leitfähigen Verbindungen 8 um Drähte handeln.

Die in Figur 4a gezeigte Ausführungsform der erfindungsgemäßen Vorrichtung 1 kann wie folgt hergestellt werden:
Zunächst wird das Substrat 4 mit den Elektroden 5, der Glasschicht 6 und den elektrisch leitfähigen Verbindungen 8 bereitgestellt. Das Substrat 4 wird in eine Spritzgussvorrichtung eingelegt. Die Spritzgussvorrichtung gibt die Abmessungen des Wandabschnitts 2 vor. Das Substrat 4 wird so in der Spritzgussvorrichtung angeordnet, dass es im Durchgangsloch 10 des Wandabschnitts 2 angeordnet sein wird. HDPE wird in fließfähiger Form in die Spritzgussvorrichtung entlang des Umfangs des Substrats 4 um das Substrat 4 herum eingespritzt und durch Abkühlen ausgehärtet. Das ausgehärtete HDPE bildet den Wandabschnitt 2, der eine Durchgangslochoberfläche 17 aufweist und durch den Spritzgussschritt fest und dicht mit dem Substrat 4 verbunden ist. Figur 4b zeigt den mit dem Substrat 4 verbundenen Wandabschnitt 2.

Im nächsten Schritt wird die Silanisierungsschicht 3 durch Plasma-Silanisieren unmittelbar auf der Durchgangslochoberfläche 17 des Wandabschnitts 2 aufgebracht. Die Silanisierungsschicht 3 wird auf einem zum Äußeren 14 eines Bioreaktors gewandten Bereich der Durchgangslochoberfläche 17 aufgebracht. Der Silanisierungsschritt wird im Wesentlichen wie für die in den Figuren 1, 2a und 2b gezeigten Ausführungsformen der erfindungsgemäßen Vorrichtung 1 beschrieben durchgeführt.

Im nächsten Schritt erfolgt das Auftragen des Klebers 7. Dazu wird der mit dem Substrat 4 verbundene Wandabschnitt 2 auf seine zum Inneren 13 eines Bioreaktors gewandte Seite auf einen Tisch gelegt. Der Kleber 7 wird von der zum Äußeren 14 eines Bioreaktors gewandten Seite des Substrats 4 her neben den elektrisch leitfähigen Verbindungen 8 aufgetragen. Durch das Auftragen und/oder durch die Schwerkraft gelangt der Kleber 7 auf die Glasschicht 6 und an die Silanisierungsschicht 3. Dabei wird nur so viel Kleber 7 aufgetragen, dass die zum Äußeren 14 eines Bioreaktors gewandten Enden der elektrisch leitfähigen Verbindungen 8 nicht von Kleber 7 bedeckt werden. Der Kleber 7 verklebt die Silanisierungsschicht 3 mit der Glasschicht 6. Dadurch wird die Vorrichtung 1 zusätzlich abgedichtet. Schließlich wird der Kleber 7 bei 65°C für 1 Stunde ausgehärtet.

Alternativ kann das Substrat 4 mit den Elektroden 5 und der Glasschicht 6, jedoch ohne die elektrisch leitfähigen Verbindungen 8 im ersten Schritt bereitgestellt werden. In diesem Fall werden die elektrisch leitfähigen Verbindungen 8 vordem Auftragen des Klebers 7, d.h. nach dem Spritzgussschritt oder nach dem Silanisierungsschritt, an die Elektroden 5 angebracht. Die elektrisch leitfähigen Verbindungen 8 werden durch Anlöten angebracht.

Figur 5 zeigt eine Querschnittsdarstellung einer bevorzugten Ausführungsform der erfindungsgemäßen Vorrichtung 1. Figur 5 entspricht im Wesentlichen der in Figur 4a gezeigten Ausführungsform, wobei die Steckdose 9 nicht gezeigt ist. Es ist ersichtlich, dass die Sensoranordnung 12 der Vorrichtung 1 einen Kunststoffkörper 15 aufweist. Der Kunststoffkörper 15 hält die elektrisch leitfähigen Verbindungen 8 zusammen. Der Kleber 7 ist bis zu der Oberfläche des Kunststoffkörpers 15, die der Glasschicht 6 zugewandt ist, aufgebracht.

Figur 6 zeigt eine Draufsicht auf die zum Inneren 13 eines Bioreaktors gewandte Oberfläche des Substrats 4. Das Substrat 4 entspricht im Wesentlichen dem Substrat 4 der in den Figuren 2b, 4a und 5 gezeigten Ausführungsformen der erfindungsgemäßen Vorrichtung 1. Auf dem Substrat 4 sind die Elektroden 5 aufgebracht, die einen Sensor bilden. Die Glasschicht 6 ist auf dem Substrat 4 zwischen den Elektroden 5 angeordnet. Es ist ersichtlich, dass die Glasschicht 6 zusätzlich auf einem Teilbereich 16 auf der dem Substrat 4 abgewandten Oberfläche einer jeden Elektrode 5 angeordnet, wobei der Teilbereich 16 den Rand 18 der dem Substrat 4 abgewandten Oberfläche einer jeden Elektrode 5 vollständig beinhaltet. Dadurch weist jede Elektrode 5 einen von der Glasschicht 6 bedeckten Teilbereich 16 auf. Jede Elektrode 5 weist in ihrer Mitte einen von der Glasschicht 6 freien Bereich auf, damit die Elektroden 5 ihre Funktion, beispielsweise für eine Leitfähigkeitsmessung, ausüben können.

Figur 7a zeigt eine Querschnittsdarstellung einer bevorzugten Ausführungsform der erfindungsgemäßen Vorrichtung 1. Figur 7a entspricht weitgehend der in Figur 4a gezeigten Ausführungsform. Unterschiedlich ist, dass in der in Figur 7a gezeigten Ausführungsform die Glasschicht 6 ausschließlich auf der zum Äußeren 14 eines Bioreaktors gewandten Oberfläche des Substrats 4 aufgebracht ist.

Die in Figur 7a gezeigte Ausführungsform der erfindungsgemäßen Vorrichtung 1 kann wie für die in Figur 4a gezeigte Ausführungsform beschrieben hergestellt werden. Figur 7b zeigt den mit dem Substrat 4 verbundenen Wandabschnitt 2.

### Bezugszeichenliste

- 1: Vorrichtung
- 2: Wandabschnitt
- 3: Silanisierungsschicht
- 4: Substrat
- 5: Elektroden
- 6: Glasschicht
- 7: Kleber
- 8: elektrisch leitfähige Verbindung
- 9: Steckdose
- 10: Durchgangsloch
- 11: Stecker
- 12: Sensoranordnung
- 13: Inneres des Behälters
- 14: Äußeres des Behälters
- 15: Kunststoffkörper
- 16: Teilbereich
- 17: Durchgangslochoberfläche
- 18: Rand

## Patentansprüche

1. Vorrichtung (1) zum Überwachen eines biologischen Prozesses in einem flüssigen Medium, mit einem Wandabschnitt (2), der eingerichtet ist, das Medium in einem Betrieb der Vorrichtung (1) zu halten, einer Silanisierungsschicht (3), einem Kleber (7), und einer Sensoranordnung (12), wobei der Wandabschnitt (2) ein Durchgangsloch (10) aufweist, die Silanisierungsschicht (3) unmittelbar auf dem Wandabschnitt (2) und vollständig um das Durchgangsloch (10) herum aufgebracht ist, die Sensoranordnung (12) einen Träger aufweist, der eine Glasschicht (6) aufweist, und der Kleber (7) die Silanisierungsschicht (3) mit der Glasschicht (6) dicht verklebt.

2. Vorrichtung (1) gemäß Anspruch 1, wobei die Glasschicht (6) der Silanisierungsschicht (3) zugewandt angeordnet ist.

3. Vorrichtung (1) gemäß Anspruch 1, wobei der Wandabschnitt (2) eine Durchgangslochoberfläche (17) aufweist, die das Durchgangsloch (10) begrenzt, wobei die Silanisierungsschicht (3) auf der Durchgangslochoberfläche (17) aufgebracht ist, und wobei die Sensoranordnung (12) in dem Durchgangsloch (10) angeordnet ist.

4. Vorrichtung (1) gemäß Anspruch 3, wobei die Glasschicht (6) im Wesentlichen senkrecht zu der Silanisierungsschicht (3) angeordnet ist.

5. Vorrichtung (1) gemäß einem der Ansprüche 1 bis 4, wobei der Kleber (7) ein Silikonkleber oder ein Epoxidharzkleber ist, wobei der Epoxidharzkleber vorzugsweise ein Zweikomponentenkleber ist.

6. Vorrichtung (1) gemäß einem der Ansprüche 1 bis 5, wobei der Wandabschnitt (2) einen Kunststoff oder ein Glas aufweist, wobei der Kunststoff vorzugsweise HDPE aufweist, und/oder wobei der Wandabschnitt (2) ein starres Bauteil ist.

7. Vorrichtung (1) gemäß einem der Ansprüche 1 bis 6, wobei der Träger aus der Glasschicht (6) besteht, wobei die Sensoranordnung (12) vorzugsweise einen optischen Sensor aufweist, der einen Detektor aufweist, der eingerichtet ist, elektromagnetische Strahlung zu detektieren, die via das Durchgangsloch (10) und die Glasschicht (6) auf den Detektor trifft.

8. Vorrichtung (1) gemäß einem der Ansprüche 1 bis 6, wobei der Träger ein Substrat (4) aufweist, auf dem die Glasschicht (6) aufgebracht ist, und die Sensoranordnung (12) mindestens einen Sensor aufweist, der auf dem Substrat (4) angeordnet ist.

9. Vorrichtung (1) gemäß Anspruch 8, wobei der Sensor einen Leitfähigkeitssensor aufweist, wobei der Leitfähigkeitssensor mindestens zwei Elektroden (5) aufweist.

10. Vorrichtung (1) gemäß Anspruch 9, wobei die Glasschicht (6) weiter zwischen den Elektroden (5) und dem Substrat (4) angeordnet ist.

11. Vorrichtung (1) gemäß Anspruch 9, wobei die Elektroden (5) auf dem Substrat (4) aufgebracht sind, wobei die Glasschicht (6) weiter zwischen den Elektroden (5) und auf einem Teilbereich (16) auf der dem Substrat (4) abgewandten Oberfläche einer jeden Elektrode (5) angeordnet ist, wobei der Teilbereich (16) den Rand (18) der dem Substrat (4) abgewandten Oberfläche einer jeden Elektrode (5) vollständig beinhaltet.

12. Bioreaktor mit einer Vorrichtung (1) gemäß einem der Ansprüche 1 bis 11 und einem Behälter, in dem in einem Bioreaktorbetrieb des Bioreaktors das Medium anzuordnen ist, wobei der Behälter den Wandabschnitt (2) aufweist, und wobei in dem Bioreaktorbetrieb der biologische Prozess durchzuführen ist.

13. Bioreaktor gemäß Anspruch 12, wobei der Träger an einer zum Äußeren (14) des Behälters gewandten Seite des Wandabschnitts (2) angebracht ist, oder wobei der Träger an einer zum Inneren (13) des Behälters gewandten Seite des Wandabschnitts (2) angebracht ist.

14. Bioreaktor gemäß Anspruch 12 oder Anspruch 13, wobei der Behälter einen Plastiksack aufweist, der vorzugsweise flexibel ist, und/oder wobei der Bioreaktor ein Einweg-Bioreaktor ist.

15. Verfahren zum Herstellen einer Vorrichtung (1) zum Überwachen eines biologischen Prozesses in einem flüssigen Medium, das Verfahren umfassend die Schritte:
a) Bereitstellen eines Wandabschnitts (2), der eingerichtet ist, das Medium in einem Betrieb der Vorrichtung (1) zu halten, und einer Silanisierungsschicht (3), wobei der Wandabschnitt (2) ein Durchgangsloch (10) aufweist und die Silanisierungsschicht (3) unmittelbar auf dem Wandabschnitt (2) und vollständig um das Durchgangsloch (10) herum aufgebracht ist,
b) Bereitstellen einer Sensoranordnung (12), wobei die Sensoranordnung (12) einen Träger aufweist, der eine Glasschicht (6) aufweist,
c) dichtes Verkleben der Silanisierungsschicht (3) mit der Glasschicht (6) mittels eines Klebers (7), und
d) Aushärten des Klebers (7).

## Claims

1. Device (1) for monitoring a biological process in a liquid medium, comprising a wall section (2) configured to hold the medium during operation of the device (1), a silanization layer (3), an adhesive (7) and a sensor arrangement (12), wherein the wall section (2) comprises a through-hole (10), the silanization layer (3) is applied directly on the wall section (2) and applied completely around the through-hole (10), the sensor arrangement (12) comprises a support comprising a glass layer (6), and the adhesive (7) tightly adhesively bonds the silanization layer (3) to the glass layer (6).

2. Device (1) according to Claim 1, wherein the glass layer (6) is arranged facing the silanization layer (3).

3. Device (1) according to Claim 1, wherein the wall section (2) comprises a through-hole surface (17) which delimits the through-hole (10), wherein the silanization layer (3) is applied on the through-hole surface (17), and wherein the sensor arrangement (12) is arranged in the through-hole (10).

4. Device (1) according to Claim 3, wherein the glass layer (6) is arranged substantially perpendicularly to the silanization layer (3).

5. Device (1) according to any of Claims 1 to 4, wherein the adhesive (7) is a silicone adhesive or an epoxy resin adhesive, wherein the epoxy resin adhesive is preferably a two-component adhesive.

6. Device (1) according to any of Claims 1 to 5, wherein the wall section (2) comprises a plastic or a glass, wherein the plastic preferably comprises HDPE, and/or wherein the wall section (2) is a rigid component.

7. Device (1) according to any of Claims 1 to 6, wherein the support consists of the glass layer (6), wherein the sensor arrangement (12) preferably comprises an optical sensor comprising a detector configured to detect electromagnetic radiation striking the detector via the through-hole (10) and the glass layer (6).

8. Device (1) according to any of Claims 1 to 6, wherein the support comprises a substrate (4) on which the glass layer (6) is applied, and the sensor arrangement (12) comprises at least one sensor which is arranged on the substrate (4).

9. Device (1) according to Claim 8, wherein the sensor comprises a conductivity sensor, wherein the conductivity sensor comprises at least two electrodes (5).

10. Device (1) according to Claim 9, wherein the glass layer (6) is furthermore arranged between the electrodes (5) and the substrate (4).

11. Device (1) according to Claim 9, wherein the electrodes (5) are applied on the substrate (4), wherein the glass layer (6) is furthermore arranged between the electrodes (5) and on a sub-region (16) on the surface of each electrode (5) that is facing away from the substrate (4), wherein the sub-region (16) completely comprises the edge (18) of the surface of each electrode (5) that is facing away from the substrate (4).

12. Bioreactor comprising a device (1) according to any of Claims 1 to 11 and a vessel in which the medium is to be arranged during bioreactor operation of the bioreactor, wherein the vessel comprises the wall section (2), and wherein the biological process is to be carried out during the bioreactor operation.

13. Bioreactor according to Claim 12, wherein the support is attached to a side of the wall section (2) that is facing the outside (14) of the vessel, or wherein the support is attached to a side of the wall section (2) that is facing the inside (13) of the vessel.

14. Bioreactor according to Claim 12 or Claim 13, wherein the vessel comprises a plastic sack which is preferably flexible, and/or wherein the bioreactor is a disposable bioreactor.

15. Method for producing a device (1) for monitoring a biological process in a liquid medium, the method comprising the steps of:
a) providing a wall section (2) configured to hold the medium during operation of the device (1), and a silanization layer (3), wherein the wall section (2) comprises a through-hole (10) and the silanization layer (3) is applied directly on the wall section (2) and applied completely around the through-hole (10),
b) providing a sensor arrangement (12), wherein the sensor arrangement (12) comprises a support comprising a glass layer (6),
c) tightly adhesively bonding the silanization layer (3) to the glass layer (6) by means of an adhesive (7), and
d) curing the adhesive (7).

## Revendications

1. Dispositif (1) de surveillance d'un procédé biologique dans un milieu liquide, avec une section de paroi (2) arrangée pour retenir le milieu pendant le fonctionnement du dispositif (1), une couche de silanisation (3), un adhésif (7), et un arrangement de capteur (12), la section de paroi (2) ayant un trou passant (10), la couche de silanisation (3) étant appliquée directement sur la section de paroi (2) et entièrement autour du trou passant (10), l'arrangement de capteur (12) ayant un support qui a une couche de verre (6), et l'adhésif (7) collant de manière étanche la couche de silanisation (3) à la couche de verre (6).

2. Dispositif (1) selon la revendication 1, dans lequel la couche de verre (6) est disposée face à la couche de silanisation (3).

3. Dispositif (1) selon la revendication 1, dans lequel la section de paroi (2) a une surface de trou passant (17) qui délimite le trou passant (10), la couche de silanisation (3) étant appliquée sur la surface de trou passant (17), et l'arrangement de capteur (12) étant disposé dans le trou passant (10).

4. Dispositif (1) selon la revendication 3, dans lequel la couche de verre (6) est essentiellement disposée perpendiculairement à la couche de silanisation (3).

5. Dispositif (1) selon l'une des revendications 1 à 4, dans lequel l'adhésif (7) est un adhésif de silicone ou un adhésif en résine époxy, l'adhésif en résine époxy étant préférablement un adhésif à deux composants.

6. Dispositif (1) selon l'une des revendications 1 à 5, dans lequel la section de paroi (2) comprend une plastique ou un verre, la plastique comprenant préférablement du HDPE, et/ou la section de paroi (2) étant un composant rigide de construction.

7. Dispositif (1) selon l'une des revendications 1 à 6, dans lequel le support est constitué par la couche de verre (6), l'arrangement de capteur (12) ayant préférablement un capteur optique ayant un détecteur arrangé pour détecter la radiation électromagnétique, qui frappe le détecteur à travers le trou passant (10) et la couche de verre (6).

8. Dispositif (1) selon l'une des revendications 1 à 6, dans lequel le support a un substrat (4) sur lequel la couche de verre (6) est appliquée et l'arrangement de capteur (12) a au moins un capteur qui est disposé sur le substrat (4).

9. Dispositif (1) selon la revendication 8, dans lequel le capteur a un capteur de conductibilité, le capteur de conductibilité ayant au moins deux électrodes (5).

10. Dispositif (1) selon la revendication 9, dans lequel la couche de verre (6) est en outre disposée entre les électrodes (5) et le substrat (4).

11. Dispositif (1) selon la revendication 9, dans lequel les électrodes (5) sont appliquées sur le substrat (4), la couche de verre (6) étant appliquée en outre entre les électrodes (5) et sur une zone partielle (16) sur la surface de chaque électrode (5) tournée à l'opposé du substrat (4), la zone partielle (16) comprenant entièrement le bord (18) de la surface de chaque électrode (5) tournée à l'opposé du substrat (4).

12. Bioréacteur avec un dispositif (1) selon l'une des revendications 1 à 11 et un boîtier dans lequel on doit placer le milieu pendant le fonctionnement du bioréacteur, le boîtier ayant la section de paroi (2), et dans lequel on doit effectuer le procédé biologique pendant le fonctionnement du bioréacteur.

13. Bioréacteur selon la revendication 12, dans lequel le support est fixé à un côté de la section de paroi (2) tourné vers l'extérieur (14) du boîtier, ou dans lequel le support est fixé à un côté de la section de paroi (2) tourné vers l'intérieur (13) du boîtier.

14. Bioréacteur selon la revendication 12 ou 13, dans lequel le boîtier comprend un sac en plastique, préférablement souple, et/ou dans lequel le bioréacteur est un bioréacteur jetable.

15. Méthode pour la production d'un dispositif (1) de surveillance d'un procédé biologique dans un milieu liquide, la méthode comprenant les étapes de:
a) fournir une section de paroi (2) arrangée pour retenir le milieu pendant le fonctionnement du dispositif (1) et une couche de silanisation (3), la section de paroi (2) ayant un trou passant (10) et la couche de silanisation (3) étant appliquée directement sur la section de paroi (2) et entièrement autour du trou passant (10),
b) fournir un arrangement de capteur (12), l'arrangement de capteur (12) ayant un support qui a une couche de verre (6),
c) coller de manière étanche la couche de silanisation (3) à la couche de verre (6) par le biais d'un adhésif (7), et
d) durcir l'adhésif (7).
